(19) Europäisches Patentamt
European Patent Office
Office européen des brevets

(11) Publication number: **0 351 857 B1**

(12) **EUROPEAN PATENT SPECIFICATION**

(45) Date of publication of patent specification: **30.11.94**   (51) Int. Cl.⁵: **G01N 33/553**, G01N 33/53

(21) Application number: **89113364.7**

(22) Date of filing: **20.07.89**

(54) **Immunoassay method using magnetic marker particles.**

(30) Priority: **20.07.88 JP 181119/88**
**15.10.88 JP 260003/88**

(43) Date of publication of application:
**24.01.90 Bulletin 90/04**

(45) Publication of the grant of the patent:
**30.11.94 Bulletin 94/48**

(84) Designated Contracting States:
**DE FR GB**

(56) References cited:

**CHEMICAL ABSTRACTS, vol. 105, no. 21, 24 November 1986, Columbus, OH (US); p. 379, no. 187154e&NUM;**

**CHEMICAL ABSTRACTS, vol. 104, no. 15, 14 April 1986, Columbus, OH (US); p. 556, no. 128110u&NUM;**

**BIOLOGICAL ABSTRACTS, vol. 86, no. 7, 1988, Biological Abstracts Inc., Philadelphia, PA (US); B. JOHNE et al., no. 70867&NUM;**

(73) Proprietor: **OLYMPUS OPTICAL CO., LTD.**
**43-2, 2-chome, Hatagaya**
**Shibuya-ku**
**Tokyo 151 (JP)**

(72) Inventor: **Nakamura, Makoto**
**2-6-22, Tsutsujigaoka**
**Akishima-shi Tokyo (JP)**
Inventor: **Tamai, Toyohiro**
**540-6, Kitano-machi**
**Hachioji-shi Tokyo (JP)**
Inventor: **Shimizu, Ryouhei**
**560-11, Kitano-machi**
**Hachioji-shi Tokyo (JP)**

(74) Representative: **KUHNEN, WACKER & PARTNER**
**Alois-Steinecker-Strasse 22**
**D-85354 Freising (DE)**

EP 0 351 857 B1

## Description

The present invention relates to an immunoassay method for measuring an antigen or antibody present in a sample by an immunoagglutination reaction.

A particle agglutination method is known as a conventional method for detecting an antigen or antibody present in a sample, on the basis of an immunoagglutination reaction. This method uses specific marker particles having an antibody or antigen which is immobilized on the surface of each particle and is able to be bonded specifically to a substance to be measured. The marker particles are mixed with a sample in a container to be immunoreacted with the antigen or antibody which is to be measured and contained in the sample, and then are allowed to collect on a predetermined wall surface (e.g., a bottom surface) of the container. The marker particles collected on the wall surface of the container have a different distribution pattern depending on whether the immunoreaction with substance to be measured has occurred or not. Therefore, positivity or negativity regarding the presence of the substance to be measured can be determined from the distribution pattern of the marker particles collected on the wall surface of the container.

A mixed agglutination method was reported by Wiener, A.S. and Herman, M.H. as another conventional method of detecting an antigen or antibody present in a sample (J. Immunol., 36,255 (1939)). This method was further developed to an extent that blood groups can be determined (Coombs, R.R.A. and Bedford, D., and Vox Sang., 5,111 (1955); Coombs, R.R.A. et al., Lacet, i, 461 (1956)). Applications for determining the blood groups on the basis of this method are exemplified in U.S.P. Nos. 4,608,246, 4,275,053, and 4,328,183.

U.S.P. No. 2,770,572 discloses a method of holding antibody which can be reacted with and bonded to an antigen in a sample on a surface of a predried solid body, thereby improving sensitivity of determination for blood groups on the surface of the solid body.

Rosenfield, R.E. et al. reported a method of causing an erythrocyte antigen to react with an antibody thereof on the surface of a solid body by utilizing the principle of the mixed agglutination method (Paris, Proc. 15th Cong. Intl. Soc. Blood Transfusion, 27 (1976)).

According to the conventional particle- and mixed-agglutination methods, precipitable particles except for the marker particles contained in the sample are precipitated, and therefore determination precision is degraded. Therefore, in order to perform accurate determination, washing and the like of the sample must be performed to eliminate the naturally precipitable particles.

Most of the conventional particle- and mixed-agglutination methods employ a technique for causing an immunoreaction in a predetermined container and collecting marker particles by natural sedimentation. It takes a long period of time to uniformly precipitate the marker particles and particularly the nonreacted particles to the bottom surface and form a distribution pattern. For example, it takes about four to six hours to determine a distribution pattern of an "anti-IgG Cell Kit" (tradename) which is sold by Olympus Optical Co. Ltd. and is used to detect an anti-platelet antibody.

In order to simply increase a sedimentation rate, the specific gravity or particle size of the marker particles may be increased. However, the specific gravity and particle size of the marker particles must be selected within the range which prevents a coarse sedimentation pattern of the particles. Therefore, a large decrease in measuring time by this method cannot be expected.

U.S.P. No. 4,297,104 discloses the following method using a centrifugal force to shorten time required for forming a sedimentation pattern of erythrocytes which is used as marker particles. According to this method, an antibody (IgG) is immobilized both on the bottom surface of a measuring container having a symmetrical axis and on the surface of each erythrocyte used as a marker particle. The antigen in the sample is reacted with the antibody immobilized on the surface of each erythrocyte in the container in advance, and an anti-IgG antibody against the antibody immobilized on the surface of the erythrocyte and on the wall surface of the container is added thereto. The first centrifugal operation is performed to cause erythrocytes as the marker particles to attach to the wall surface of the container through the added anti-IgG. After the first centrifugal operation is interrupted, the second centrifugal operation is performed to collect the erythrocytes nonreacted with the antigen in the sample on the bottom surface of the container, thereby forming a sedimentation pattern.

The centrifugal method is very effective to accelerate agglutination by an immunoreaction and sedimention of the marker particles. However, the centrifugal operations pose the following problems.

First, it is difficult to smoothly handle the measuring container in a series of operations, ranging from sampling to carrying out the reaction, determination, waste disposal, and the like. In particular, it is not easy to align the symmetrical axis of the measuring container with a direction of centrifugal force.

2

Second, it is difficult to analyze different items. Analysis of different items requires different sedimentation conditions. It is very difficult to set different degrees of centrifugal forces and different centrifugal times for respective samples in the same centrifugal cycle. In order to analyze a plurality of items, therefore, a plurality of centrifugal operations must be performed under different conditions for respective analysis items, or a plurality of centrifugal machines set in different conditions for respective analysis items must be used.

Third, since a centrifugal machine must be used in an automatic analysis process, the apparatus becomes bulky.

In Japanese Kokai Tokkyo Koho JP 61,128,168 there are disclosed agglutination tests in which magnetic as well as labeled insoluble carrier particles are sensitized with antibodies or antigens and are allowed to react with a sample containing antigens or antibodies, whereby the magnetic particles are removed under the influence of a magnetic field, and the label in the agglutinated particles is quantitatively measured for the detection of the antigens or antibodies in the sample.

It is a first object of the present invention to improve an immunoassay method by a particle agglutination method, and particularly, to shorten time required for determining an agglutination reaction without using marker particles having a large specific gravity or a large particle size and without using a centrifugal operation.

It is a second object of the present invention to provide an immunoassay method of performing high-precision measurements of a sample containing a precipitable substance other than a substance to be measured, without being interfered by the precipitable substance.

The objects described above are achieved by an immunoassay method as described in claim 1 and an apparatus as described in claim 10.

According to the present invention, it is preferable that a given substance which can be bonded specifically to or compete with the substance to be measured is also immobilized, in advance, on the inner surface of the predetermined wall surface region of the measuring container.

According to the method of the present invention, the magnetic marker particles containing a magnetic material are used as marker for the substance to be measured. A sedimentation rate of the magnetic marker particles is increased by utilizing an appropriate magnet. The sedimentation pattern of the marker particles can be formed within a very short period of time as compared with the conventional particle agglutination method. Therefore, time required for determining the sedimentation pattern can be shortened.

In addition, since sedimentation of only the magnetic marker particles can be accelerated, the sedimentation pattern of the magnetic marker particles can be formed before other precipitable particles precipitate. Therefore, high-sensitivity measurement can be performed without eliminating naturally precipitable particles other than the substance to be measured beforehand.

This invention can be more fully understood from the following detailed description when taken in conjunction with the accompanying drawings, in which:

Figs. 1A and 1B are a plan view and a side view, respectively, showing an arrangement of magnets when a microplate is used as a measuring container according to an embodiment of the present invention;

Figs. 2A and 2B are views showing a positive sedimentation distribution pattern obtained by using magnetic marker particles which can be bonded specifically to the substance to be measured;

Figs. 3A and 3B are views showing a negative sedimentation distribution pattern obtained by using magnetic marker particles which can be bonded specifically to the substance to be measured;

Fig. 4A is a view showing a first positive sedimentation distribution pattern obtained by using magnetic marker particles, which can be bonded specifically to the substance to be measured, and a measuring container having the given substance which is immobilized on the inner surface of the container and can be bonded specifically to the substance to be measured;

Fig. 4B is a view showing a second positive sedimentation distribution pattern obtained by using magnetic marker particles, which can be bonded specifically to the substance to be measured, and a measuring container having the given substance which is immobilized on the inner surface of the container and can be bonded specifically to the substance to be measured;

Fig. 4C is a view showing a negative sedimentation distribution pattern obtained by using magnetic marker particles, which can be bonded specifically to the substance to be measured, and a measuring container having the given substance which is immobilized on the inner surface of the container and can be bonded specifically to the substance to be measured;

Fig. 5 is a perspective view showing a measuring container and a magnet according to another embodiment of the present invention;

Fig. 6 is a reaction accelerating apparatus suitably used in the present invention; and

Fig. 7 is another reaction accelerating apparatus suitably used in the present invention.

An embodiment of the method of the present invention will be described in detail below.

A substance to be measured according to the present invention can be an antigen substance such as viruses, bacteria, or various proteins, and an antibody against the antigen substance. The substance to be measured may be a soluble or insoluble substance.

A marker substance (e.g., a specific antibody or antigen) which can be bonded specifically to or compete with a substance to be measured is immobilized on the surface of each known magnetic particle containing a magnetic material to prepare magnetic marker particles. These magnetic marker particles can be bonded specifically to or compete with the substance to be measured respectively. Examples of the magnetic particles are "DYNABEAS M-450" (tradename) available from DYNAL Corp. and magnetic latex beads "estapor" (tradename) available from RHONE-POULENC Corp. Gelatin particles containing a magnetic material, as disclosed in Japanese Patent Disclosure (Kokai) No. 59-19516, may be used. In addition, cells such as erythrocytes containing a incorporated ferromagnetic material such as an iron powder may also be used. Various known methods may be used as a method of immobilizing the specific antibody or antigen on the surfaces of these magnetic particles.

The sample to be measured is mixed and immuno-reacted with the magnetic marker particles.

The reaction solution is then stored in a predetermined measuring container. Alternatively, the above reaction may be performed in this measuring container. The type of measuring container is not limited to a specific one. However, a measuring container preferably has an inclined wall surface such as a semispherical or conical surface to collect the magnetic marker particles in a predetermined region when the magnetic marker particles move along the wall surface of the container in a predetermined direction. The inclined wall surface of the measuring container may be a side surface but is preferably a bottom surface. An example of the preferable measuring container is a test tube or microplate having, e.g., a semispherical or conical bottom surface.

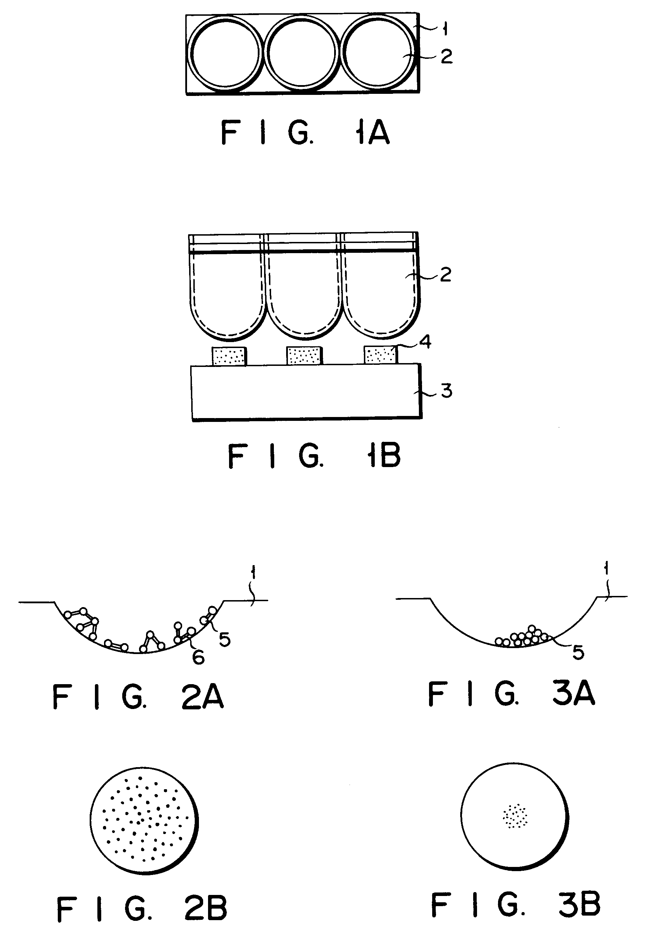

A magnet is located near at least the inclined wall surface region of the outer wall surface of the container. For example, as shown in Figs. 1A and 1B, when a microplate 1 is used as a measuring container, magnets 4 placed on a support member 3 are located near the semispherical bottom surfaces of wells 2, respectively. Fig. 1A is a plan view showing the microplate and Fig. 1B is a side view showing the relationship between the magnets 3 and the wells 2. The preparation of the magnetic marker particles and immuno-reaction between the magnetic marker particles and a sample may be performed in the measuring container. When the magnets 4 are located near the bottom surface of the measuring container, the magnetic marker particles are attracted toward the magnets 4 and can be precipitated at a rate higher than that of natural sedimentation.

The distribution pattern of the precipitated magnetic marker particles varies depending on the presence or absence of the substance to be measured in the sample. The distribution pattern also varies depending on whether the magnetic marker particles can be bonded specifically to or compete with the substance to be measured.

First, described below is the case in which a magnetic marker particles which can be bonded specifically to a substance is used. In this case, when the substance to be measured is present in a sample, a plurality of magnetic marker particles 5 are bonded to a substance 6 to be measured to form an agglutinate, as shown in Fig. 2A. For this reason, the magnetic marker particles 5 cannot move further. As shown in Fig. 2B, therefore, a distribution pattern of the magnetic marker particles 5 uniformly spreads on the spherical wall surface of the measuring container 1 (positivity). On the other hand, when a substance to be measured is not present in a sample, an agglutinate is not formed. Therefore, the magnetic marker particles precipitated on the spherical wall surface of the container move downward along the spherical wall surface and collect on the lowest portion of the spherical wall surface, as shown in Fig. 3A. As a result, the magnetic marker particles 5 collected at the central portion of the spherical wall surface (negativity) form a distribution pattern, as shown in Fig. 3B. Therefore, by observing the distribution pattern of the precipitated magnetic marker particles with a naked eye or an optical measuring unit, it is possible to determine the presence or absence of the substance to be measured and to quantitatively measure the substance to be measured.

When the magnetic marker particles competing with the substance to be measured (e.g., the immobilized substance is the same as the substance to be measured) is used, a crosslinking substance which bonds to both the substance to be measured and the magnetic marker particles must be added in a sample in addition to the magnetic marker particles. If a substance to be measured is not present in a sample, the crosslinking substance is bonded to the magnetic marker particles. Therefore, a plurality of magnetic marker particles are bonded and agglutinated to each other through the crosslinking substance. Therefore, the resultant sedimentation pattern is the same as shown in Figs. 2A and 2B (negativity). To the contrary, when the substance to be measured is present in a sample in a large amount, the crosslinking substance is

4

bonded to the substance to be measured. For this reason, agglutination of the magnetic marker particles through the crosslinking substance slightly or does not occur. In this case, the sedimentation pattern is the same as shown in Figs. 3A and 3B (positivity). This method is known as an agglutination inhibition reaction. When measurement is performed by the agglutination inhibition reaction, the distribution patterns of the precipitated magnetic marker particles are opposite to those obtained when magnetic marker particles which can be bonded to the substance to be measured is used.

According to the present invention, the given substance which can be bonded to or compete with the substance to be measured in the sample may also be immobilized on the wall surface of the measuring container.

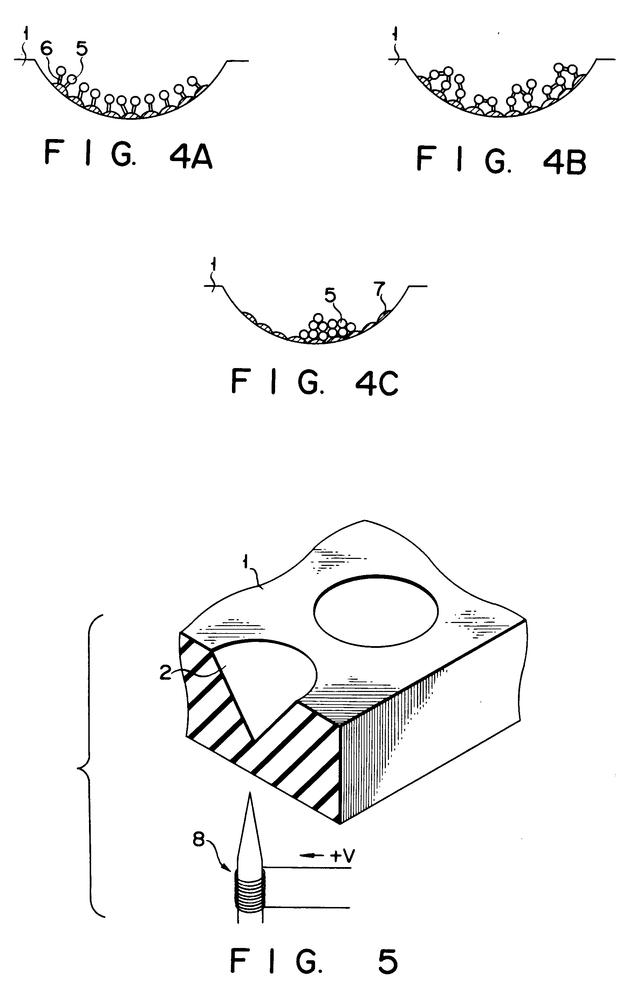

Described below with reference to Fig. 4A to 4C is a case wherein a trapping substance 7 which can specifically bond to a substance 6 to be measured in a sample is used as the given substance immobilized on the wall surface of container 1, and magnetic marker particles 5 which can be bonded specifically to the substance to be measured is used.

A sample is set and incubated, in the measuring container 1. The sample is removed, and then the container is washed. A substance 6 to be measured in the sample is bonded to the trapping substance 7 and trapped thereon. A solution of the magnetic marker particles is poured in the container 1, and a magnetic field from the magnets 4 is applied to precipitate the magnetic marker particles 5. The precipitated magnetic marker particles 5 are bonded to the substance 6 trapped on the trapping substance 7, as shown in Fig. 4A, and do not move downward along the wall surface of the container. The precipitated magnetic marker particles 5 have a uniform distribution pattern (positivity), as shown in Fig. 2B. To the contrary, when the substance 6 is not present in the sample, the precipitated magnetic marker particles 5 are not bonded to the wall surface of the container. Therefore, the magnetic marker particles 5 move to the central portion of the wall surface of the container to form a distribution pattern (negativity) concentrated at the central portion of the spherical wall surface, as shown in Fig. 3B. In this case, since the sample is removed from the container 1, the container 1 is washed, and then the magnetic marker particles 5 are added, formation of the distribution pattern of the magnetic particles is not interfered by other components in the sample. Therefore, it is possible to perform high-sensitivity measurement. Since the magnetic particles are bonded to the wall surface of the container in the positive reaction, positivity and negativity can be more accurately determined, which contributes to an improvement of detection sensitivity.

Instead of removing the sample and washing the measuring container 1, after the magnetic marker particles are added to the sample, the incubated sample contained in the measuring container may be diluted. This can also improve the measuring sensitivity. In this case, the agglutination pattern of the magnetic marker particles in the positive reaction is a state not shown in Fig. 4A but in Fig. 4B.

When the trapping substance is immobilized on the wall surface of the container 1 and the magnetic marker particles which compete with the substance to be measured is used, the substance 6 to be measured and the magnetic marker particles 5 compete with each other with respect to the trapping substance 7 of the wall surface of the container. For this reason, the positive and negative distribution patterns are reversed. That is, the trapping substance 7 is bonded to the substance 6 to be measured and is saturated in a positive reaction. Therefore, the magnetic marker particles 5 are not trapped, and the distribution pattern (Fig. 4C) concentrated at the central portion of the spherical wall surface is obtained. To the contrary, the trapping substance 7 on the wall surface of the container is not saturated in a negative reaction. In this case, the magnetic marker particles 5 are bonded to the trapping substance 7, thereby forming a uniform distribution pattern as shown in Fig. 4A.

The substance (e.g., the same substance as the substance 6 to be measured) which compete with the substance 6 to be measured may be immobilized on the wall surface of the container 1. In this case, the distribution patterns of the precipitated magnetic marker particles 5 are opposite to those wherein the trapping substance which can specifically bond to the substance 6 is immobilized on the wall surface of the container. A mechanism for forming the reverse distribution patterns will not be described.

As is apparent from the above description, according to the present invention, by using the magnetic marker particles and action of the magnet, it is possible to accelerate sedimentation of the marker particles. Even if the size or specific gravity of the marker particles is not increased, or the centrifugal operation is not employed, the time for forming the sedimentation pattern of the magnetic particles can be greatly shortened. Therefore, coarseness of the sedimentation pattern can be prevented, disadvantages posed by the centrifugal operations can be prevented, and the measuring time can be greatly shortened.

Since sedimentation of only the magnetic marker particles can be selectively accelerated according to the present invention, measurement can be accurately performed without being interfered by an insoluble substance or a precipitable substance other than that to be measured in the sample. More specifically, it is possible to form a sedimentation pattern of the magnetic marker particles before the insoluble or precipitable substance other than that to be measured is precipitated, by applying a magnetic field from the magnet prior to or in the initial stage or sedimentation of the insoluble or precipitable substance. Further, even if a sample is a solution having a high viscosity or a solution having a larger specific gravity than that of the magnetic marker particles, sedimentation of the magnetic marker particles can be selectively accelerated to perform accurate measurements.

Since the magnetic marker particles are forcibly moved by the action of the magnet according to the present invention, the magnetic marker particles can be moved in a direction different from the sedimentation direction of the insoluble or precipitable substance other than that to be measured. For example, when a test tube is used as a measuring container, a magnet can be located near its side wall to collect the magnetic marker particles on the side wall, while precipitating the insoluble or precipitable substance other than that to be measured by natural sedimentation. According to this method, a sedimentation distribution pattern of the magnetic marker particles can be formed without being adversely affected by the precipitable substance other than that to be measured.

Since the measurement can be performed without being interfered by sedimentation of the substance other than that to be measured, the method of the present invention can be effectively applied to samples (e.g., whole blood or chylonicronemia) containing precipitable or insoluble substances other than that to be measured. That is, without separating a precipitable or insoluble substance other than that to be measured from a sample, good measurement results can be obtained.

Moreover, the method of the present invention can be simultaneously performed with the conventional measurement using nonmagnetic marker particles. For example, measurements using magnetic marker particles are performed in some wells of a microplate, while measurements using nonmagnetic marker particles are performed in other wells of the same microplate. In other words, even if the magnet applies a magnetic field to accelerate sedimentation of the magnetic marker particles in some wells, the magnetic field does not affect the nonmagnetic marker particles in other wells. Therefore, the present invention is very effective to simultaneously perform measurements of many items.

The magnet used in the present invention is not limited to any specific type of magnet, and may be a permanent magnet or an electromagnet. The strength of the magnet can be appropriately selected in accordance with a substance to be measured and the like. The magnet need not be limited to that of disk shape, but may have any shape. A needle-like magnet 8 having a sharp tip, as shown in Fig. 5, is most preferable due to the following reason. As shown in Fig. 3A or 4C, when the magnetic marker particles 5 are concentrated at the center of the bottom surface of the measuring container, the magnetic marker particles 5 can be gathered together in a narrower area by using the needle-like magnet 8. Therefore, a difference between the positive and negative patterns can become clearer, and measuring sensitivity can be improved. The wall surface of the well 2 having a conical shape (Fig. 5) is preferable to that having a spherical shape.

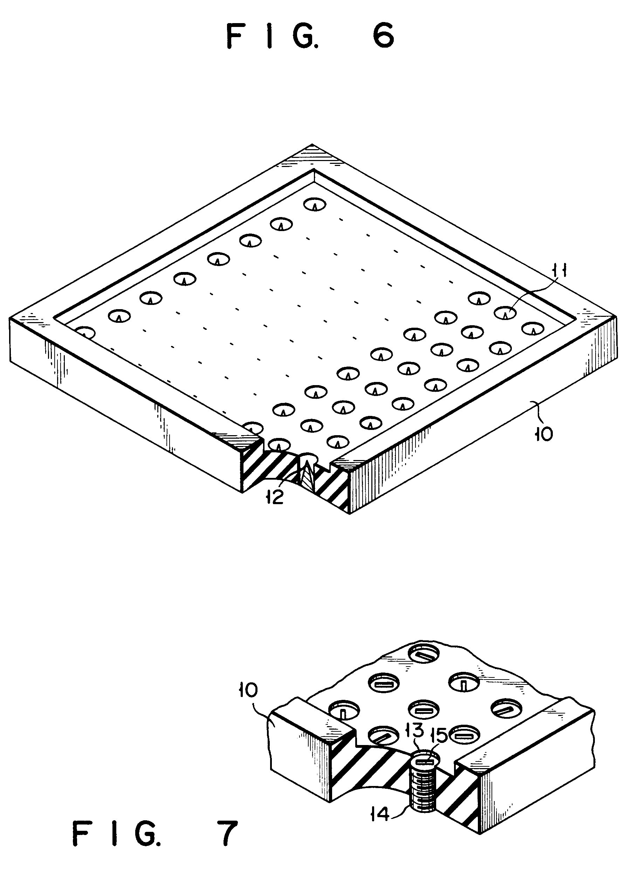

When a microplate having a large number of wells 2 is used as a measuring container, a reaction accelerating apparatus shown in Fig. 6 is preferably used. The reaction accelerating apparatus has a supporting base having raised flange at the edge to support a microplate having 8 × 12 wells without causing positional errors. Circular recesses 11 are formed in the supporting base 10 at positions respectively corresponding to the wells of the microplate. Needle-like ferrite magnets 12 (magnetic flux density: 2,200 gauss) each having a conical shape are fitted in the recesses 11. When this reaction accelerating apparatus is used, the needle-like magnets 12 can be automatically located at the central portions of the bottom surfaces of the wells, respectively, when the microplate is placed on the supporting base 10.

Fig. 7 shows another reaction accelerating apparatus which can be preferably used. In the apparatus, circular recesses 13 having female threads are formed in the supporting base 10 at positions respectively corresponding to the wells of the microplate. Cylindrical magnets 14 having male threads are respectively screwed into the recesses 14. The screwing of the cylindrical magnet 14 is preformed by rotating the magnet 14. In order to rotating the magnet 14 by using a screw driver, a groove 15 is formed on the upper end face of the magnet 14. The groove 15 may be either minus shaped (-) or plus shaped (+). The upper end of the magnet 14 may be N-pole or S-pole. In the apparatus shown in Fig. 7, it is easy to attain fine adjustment of the hight of the upper end face of the magnet in the recess 13 by controlling the rotation of the magnet 14.

According to the present invention, the step of applying a magnetic field from each magnet to accelerate sedimentation of the magnetic marker particles may be performed while the measuring container is kept stationary or transported.

The present invention will be described in detail by way of its examples below.

Example 1

Screening of Irregular Antibody by Antiglobulin Test (Coomb's Test)

[A] Preparation of O-cells Coated Microplate

A microplate with a U-shaped bottom (Manufacture No. 464,394 available from NUNC Corp.) was prepared. 100 $\mu\ell$ of a wheat germ lectin solution (WGA available from Seikagaku Kogyo K.K.) prepared to have a concentration of 10 $\mu$g/m$\ell$ by using a 0.01M phosphate buffer solution (PBS) of pH 7.0 were added to each well of the microplate and were treated at room temperature for 30 minutes. Each well was washed five times by using 300 $\mu\ell$ of the 0.01M PBS of pH 7.0 each time. The microplate was then dried at room temperature to obtain a WGA-treated plate.

A surgisurge screen (Manufacture No. 3SS837 available from Ortho Corp.) as human Group O erythrocytes having blood group antigens shown in Table 1 was diluted to 0.3% with a physiological saline. The diluted surgiscreen was added to the the WGA-treated plate in an amount of 25 $\mu\ell$ per well, and the microplate was left to stand at room temperature to adsorb the surgiscreen to the surface of each well of the microplate. Each well was washed three times by using 200 $\mu\ell$ (each time) of 0.01M PBS of pH 7.0 containing a 0.1% bovine serum alubmin.

## Table 1

### Doner No. 41901; Genetype: $R_2R_2$

| D | C | E | $\bar{c}$ | $\bar{e}$ | f | K | $\bar{k}$ | Fy$^a$ | Fy$^b$ | JK$^a$ | JK$^b$ | Xg$^a$ |
|---|---|---|---|---|---|---|---|---|---|---|---|---|
| + | − | + | + | − | − | − | + | + | + | − | + | + |

| Le$^a$ | Le$^b$ | S | $\bar{s}$ | M | N | P1 |
|---|---|---|---|---|---|---|
| − | + | + | − | + | − | + |

**Note) +: antigen is present;**
**−: antigen is not present**

[B] Preparation of Anti-Human IgG Sensitized Latex Beads

A solution (concentration: 0.4%) of dark brown magnetic latex beads (tradename: estapor available from RHONE-POULENC Corp.; manufacture No. LMP233) each having a particle size of 1 $\mu$ was prepared by using a 0.1M glycine-sodium hydroxide buffer solution of pH 8.3 (to be referred to as a glycine buffer solution hereinafter). A solution (concentration: 1 $\mu$g/m$\ell$) of an anti-human IgG (manufacture No. 0601-0081 available from Capple Corp.) was prepared by using the same glycine buffer solution. 2 m$\ell$ of the former solution was mixed with 2 m$\ell$ of the latter solution and reacted therewith at 37°C for an hour to sensitize the magnetic latex beads with the anti-human IgG. The sensitized latex beads were washed three times with 2 m$\ell$ of the 0.1M glycine buffer solution of pH 8.3 containing a 1% BSA and was then reacted in the same 2-m$\ell$ buffer solution at 37°C for 30 minutes, thereby performing blocking for inhibiting a nonspecific agglutination. The latex beads were then washed three times with 2 m$\ell$ (each time) of the 0.01M PBS of pH 7.0 containing a 0.01% sodium N-lauroyl sarcosinate (surfactant available from Nikko Chemicals K.K.) and a 0.1% BSA. The resultant latex was preserved in the same 4-m$\ell$ PBS.

[C] Detection of Irregular Antibody

60 $\mu\ell$ of an LISS solution and 30 $\mu\ell$ of any one of antiserums (available from Ortho Corp.), i.e., anti-$\bar{k}$, anti-K, anti-Fy$^a$, and anti-$\bar{s}$ were added to each well of the O-cells coated microplate prepared in [A] and were reacted at 37°C for 30 minutes. At the same time, 30 m$\ell$ of the 0.01M PBS were added as a negative control to one well in place of the antiserum and were reacted similarly.

Each well was washed four times with 200 $\mu\ell$ (each time) of the 0.01M PBS of pH 7.0, and 25 $\mu\ell$ of the anti-human IgG magnetic latex beads were added to each well. Subsequently, as shown in Figs. 2A and 2B, the bottoms of the wells 7 were respectively located above the disc-like ferrite magnets 5, respectively,

7

and a further reaction was performed at room temperature for 2 minutes while a magnetic field was applied to the bottom surfaces of the wells 7 in the vertical direction. The diameter of each ferrite magnet 5 was 8 mm, its thickness was 3 mm, and a magnetic flux density was 2,000 gauss.

With the above operations, the anti-human IgG latex beads were precipitated on the bottom surface of each well 7. When the anti-human IgG magnetic latex beads were uniformly distributed, this distribution was determined as a positive reaction. When the anti-human IgG magnetic latex beads were concentrated at the center of each well, this distribution is determined as a negative reaction. Results are shown in Table 2. The results were matched with the specific antigens of the erythrocyte in Table 1.

Table 2

| Antiserum | K | $\bar{k}$ | Fy$^2$ | $\bar{s}$ | PBS |
|---|---|---|---|---|---|
| Reaction Pattern | - | + | + | - | - |

In the above detection, since sedimentation of the anti-human IgG magnetic latex beads as the magnetic marker particles was accelerated by the magnets, the time required for determining the distribution patterns was reduced to about 5 to 20% of the time required in the conventional particle agglutination method.

As can be understood from the above description, Example 1 was performed by the method described with reference to Figs. 4A and 4C. That is, the surgiscreen as the trapping substance 7 was immobilized on the surface of each well. The well was washed after the sample was reacted, thereby obtaining a stable, clear sedimentation distribution pattern of the magnetic latex beads and a good detection sensitivity.

The following comparative experiment was performed to compare the detection sensitivity of the present invention with that of the conventional method and to evaluate the detection sensitivity of the present invention.

[D] Sensitivity Comparison With Conventional Method

(a) Group O erythrocytes which can be reacted respectively with the twelve commercially available irregular antibodies shown in Table 3 were selected from the surge screen (manufacture No. 3SS119 available from Ortho Corp.) including three types of Group O erythrocytes (Nos. 1 to 3).

Table 3

| Irregular Antibody | Manufacture Lot No. | Group O Erythrocyte No. Selected from Surgiscreen (3SS119) |
|---|---|---|
| E | Cooper Biomedical 753 | 2 |
| C | Cooper Biomedical 653 | 1 |
| e | Cooper Biomedical 963 | 3 |
| $\bar{c}$ | Cooper Biomedical 848 | 2 |
| K | Ortho KCO32E2 | 3 |
| k | Ortho LK139A | 3 |
| Fy$^a$ | Ortho FA173B | 3 |
| Fy$^b$ | Cooper Biomedical 474 | 2 |
| JK$^a$ | Cooper Biomedical 2041 | 3 |
| JK$^b$ | Cooper Biomedical 2610 | 2 |
| S | Ortho SS151A | 3 |
| s | Ortho LS147B | 2 |

Each surgiscreen was diluted with a physiological saline to prepare a 0.7% suspension. The diluted suspension was distributed to a WGA-treated plate in an amount of 25 $\mu\ell$ per well and was left to stand at room temperature for 10 minutes. Each well was washed with the physiological saline to prepare a O-cells coated microplate which can be reacted with the respective antibody.

The twelve irregular antibodies shown in Table 3 were sequentially diluted two times, four times, 16 times,... with a physiological saline. 66.7 $\mu\ell$ of an LISS solution were added with respect to 100 $\mu\ell$ of

8

the diluted solution, thereby preparing a series of multiple-diluted samples of each irregular antibody.

The following experiment was performed using the series of diluted samples. Diluted samples having different concentrations were distributed to the wells of the solid-phase plate in an amount of 25 $\mu\ell$ per well, and were incubated at 37° for 10 minutes. The incubated samples were washed with a physiological saline six times. Subsequently, the magnetic latex beads sensitized by the anti-human IgG antibody in [B] were distributed to the wells in an amount of 25 $\mu\ell$/well and were left to stand on the magnet for 2 minutes following the same procedures as described above, thereby forming sedimentation patterns. When the precipitated anti-human IgG magnetic latex beads were uniformly spread on the entire surface of each well, its distribution was determined as strong positivity (+ +). When the beads were almost uniformly spread on the entire surface, its distribution is determined as positivity (+). When the beads were slightly spread on the surface of each well, its distribution was determined as weak positivity (±). When the beads were concentrated at the central portion of the bottom of each well, its distribution is determined as negativity (-). Results are shown in Table 4.

(b) In order to exemplify a comparative example for evaluating measurement by the method of the present invention, a measurement by the conventional method on the basis of the Assay method proposed by Ortho Corp. was performed as follows.

The twelve irregular antibodies were sequentially diluted in a multiple series as described above, 30 $\mu\ell$ of an OAES (Ortho AES200) were added to 60 $\mu\ell$ of each diluted antibody to prepare each sample. 30 $\mu\ell$ of a reactive surge screen shown in Table 2 were added to each sample and were incubated at 30°C for 15 minutes. Each sample was washed three times with 3 m$\ell$ of a physiological saline each time. 60 $\mu\ell$ of a Coomb's serum (Manufacture No. HH858HI-1 available from Ortho Corp.) were added to each sample. Each sample was then subjected to a centrifugal operation at 1,200 rpm for 30 seconds. Positivity and negativity of the samples were determined based on sedimentation patterns in accordance with the standards described above. Results are summarized in Table 4.

It is apparent from the results of Table 4 that the detection sensitivity of the present invention is higher than that of the conventional method.

## Table 4

| Method of Present Invention | | | | | | | Irregular Antibody | Conventional Method | | | | | | |
|---|---|---|---|---|---|---|---|---|---|---|---|---|---|---|
| 1/128 | 1/64 | 1/32 | 1/16 | 1/8 | 1/4 | 1/2 | | 1/2 | 1/4 | 1/8 | 1/16 | 1/32 | 1/64 | 1/128 |
| ++ | ++ | ++ | ++ | ++ | ++ | ++ | E | ++ | ++ | ++ | ++ | ++ | ++ | + |
| ± | ++ | ++ | ++ | ++ | ++ | ++ | C | ++ | ++ | ++ | ± | ± | − | − |
| ± | ++ | ++ | ++ | ++ | ++ | ++ | e | ++ | ++ | ++ | ++ | + | ± | ± |
| ++ | ++ | ++ | ++ | ++ | ++ | ++ | $\bar{c}$ | ++ | ++ | ++ | ++ | + | + | ± |
| − | ± | ++ | ++ | ++ | ++ | ++ | K | ++ | ++ | ++ | + | ± | − | − |
| − | + | ++ | ++ | ++ | ++ | ++ | $\bar{k}$ | ++ | ++ | + | + | ± | − | − |
| ± | ± | ++ | ++ | ++ | ++ | ++ | $Fy^a$ | ++ | ++ | ++ | + | ± | − | − |
| + | ++ | ++ | ++ | ++ | ++ | ++ | $Fy^b$ | ++ | + | + | ± | − | − | − |
| − | ± | ++ | ++ | ++ | ++ | ++ | $Jk^a$ | ++ | ++ | ++ | + | ± | − | − |
| − | ± | ++ | ++ | ++ | ++ | ++ | $Jk^b$ | ++ | ++ | + | ± | ± | − | − |
| − | ± | ++ | ++ | ++ | ++ | ++ | S | ++ | ++ | ++ | + | ± | − | − |
| − | ± | ++ | ++ | ++ | ++ | ++ | $\bar{s}$ | ++ | ++ | ± | − | − | − | − |

EP 0 351 857 B1

Example 2

Direct Antiglobulin Test for Autoantibody Detection

[A] Preparation of Erythrocyte Coated Microplate

A WGA-treated plate was prepared following the same procedures as in Example 1. Erythrocytes separated from blood samples of interest which were added with an anticoagulant were washed with a physiological saline and were suspended in the physiological saline, thereby preparing a 0.3% erythrocyte suspension. The erythrocyte suspension was distributed to the wells of the WGA-treated plate in an amount of 25 $\mu\ell$/well and was left to stand at room temperature for 10 minutes. Each sample was washed once with 200 $\mu\ell$ of a 0.01M PBS of pH 7.0 containing a 0.2% gelatin.

[B] Preparation of Anti-Rabbit IgG Sensitized Magnetic Latex Beads

Magnetic latex beads sensitized with an anti-rabbit IgG (manufacture No. 50-0115-16 available from Kirkegaard & Perry Laboratories Inc.) were prepared following the same procedures as in Example 1 except that the anti-rabbit IgG was used in place of the anti-human IgG used in Example 1.

[C] Detection of Autoantibody

A rabbit-originated Coomb's serum (Ortho Corp.) was distributed to the wells of the erythrocyte-cells coated microplate prepared in [A] in an amount of 25 $\mu\ell$ per well and was reacted at room temperature for 10 minutes. Each well was washed three times with 200 $\mu\ell$ of a 0.01M PBS of pH 7.0 each time, and the anti-rabbit IgG magnetic latex beads prepared in [B] was added to each well in an amount of 25 $\mu\ell$ per well. A magnetic field from the magnets was applied to the wells in the same manner as in Example 1, so that the anti-rabbit IgG magnetic latex beads were precipitated to form distribution patterns on the bottom surfaces of the wells, thereby determining positivity and negativity of the distribution patterns.

In Example 2, the time required for determining the distribution patterns can be reduced to about 5 ~ 20% of the time required for the conventional method.

Since the method described with reference to Figs. 4A and 4C is used in the same manner as in Example 1, a high detection sensitivity can be obtained.

Example 3

HBs Antigen Test (No. 1)

[A] Preparation of Anti-HBs Antibody Coated Microplate

A rabbit anti-HBs antibody purified by an affinity column was buffered by a 0.01M phosphate buffer solution (PBS) of pH 7 to have a concentration of 10 $\mu$g/m$\ell$. The anti-HBs antibody was added to wells of a microplate having a U-shaped bottom (manufacture No. 464394 available from NUNC Corp.) in an amount of 100 $\mu\ell$/well and was incubated at 37°C for an hour and adsorbed on the surface of the wells. Each well was washed three times with 250 $\mu\ell$ of a 0.01M PBS of pH 7.0 each time. 200 $\mu\ell$ of a 0.01M PBS of pH 7.0 containing a 0.2% bovine serum albumin (BSA) were added to each well and were reacted at 37°C for an hour to perform blocking. In addition, each well was washed three times with 250 $\mu\ell$/well (each time) of a 0.01M PBS of pH 7.0 containing a 0.05% Tween 20. The microplate was then dried at room temperature and preserved at 4°C.

[B] Preparation of Anti-HBs Antibody Sensitized Magnetic Latex Beads

A magnetic latex bead solution (concentration: 0.4%) as in Example 1 was prepared by using a 0.1M glycine buffer solution of pH 8.3. By using the same glycine buffer solution, an anti-HBs antibody solution (concentration: 2.5 $\mu$g/m$\ell$) was prepared. 2 m$\ell$ of the magnetic latex bead solution were mixed with 2 m$\ell$ of the anti-HBs antibody solution and were reacted therewith at 37°C for an hour, thereby sensitizing the magnetic latex beads with the HBs antibody. The sensitized magnetic latex beads were washed three times with 2 m$\ell$ (each time) of a 0.1M glycine buffer solution of pH 8.3 containing a 1% BSA. The latex beads were reacted in the same 2-m$\ell$ buffer solution at 37°C for 30 minutes to perform blocking. In addition, the

EP 0 351 857 B1

beads were washed three times with 2 mℓ (each time) of a 0.01M PBS containing a 0.05% Tween® 20 and a 0.1% BSA. 4 mℓ of the same PBS were added to the beads, and the beads were preserved in the PBS.

[C] Detection of HBs Antigen

An HBs antigen positive serum as a sample was added to each well of the anti-HBs antibody coated microplate without dilution in an amount of 25 μℓ/well and was reacted therewith at room temperature for 15 minutes. At the same time, 25 μℓ of an HBs antigen negative serum were added as a control to one well without dilution and were reacted with the antibody. The added serums were removed, and each well was washed once with 200 μℓ of a 0.01M PBS of pH 7.5. The anti-HBs antibody sensitized magnetic latex beads prepared in [B] were added to the wells in an amount of 25 μℓ/well. The magnetic latex beads were precipitated by using a magnet to form distributed patterns following the same procedures as in Example 1.

The time required for determining the distribution patterns in Example 3 was reduced to about 5 ~ 20% of the time required for the conventional method.

Since the method described with reference to Figs. 4A and 4C is used as in Example 1, a high detection sensitivity can be obtained.

Example 4

HBs Antigen Test (No. 2)

[A] Preparation of Anti-HBs Antibody Coated Microplate

A microplate coated with an anti-HBs antibody was prepared following the same procedures as in Example 3.

[B] Preparation of Anti-HBs Antibody Sensitized Latex Beads

Magnetic latex beads sensitized with anti-HBs antibody were prepared following the same procedures as in Example 3.

[C] Detection of HBs Antigen

An HBs antigen positive serum as a sample was added to each well of the anti-HBs antibody coated microplate prepared in [A] in an amount of 25 μℓ/well without dilution in the same manner as in Example 3. 25 μℓ of an HBs antigen negative serum were added to one well as a control without dilution. Subsequently, the anti-HBs antibody sensitized magnetic latex beads prepared in [B] was added to each well in an amount of 25 μℓ/well and was reacted therewith at room temperature for 15 minutes.

A 0.01M PBS of pH 7.5 containing a 0.05% Tween® 20 and a 0.1% BSA was added to each well in an amount of 150 μℓ/well. A magnetic field was applied from magnets to the bottom surfaces of the wells to precipitate the magnetic latex beads on the bottom surfaces of the wells, thereby forming distribution patterns in the same manner as in Example 1.

The time required for determining the distribution patterns can be reduced to about 5 ~ 20% of the time required in the conventional method.

Unlike Example 3, neither the sample was wasted nor each well was washed after the samples were reacted with the magnetic marker particles in Example 3. However, since the samples are diluted after they are reacted with the magnetic marker particles according to the method described with reference to Figs. 4B and 4C, the problem of a nonspecific agglutination reaction occurring in a sample serum having a high concentration can be easily prevented without degrading operability. The problem of false positive caused by the nonspecific agglutination reaction can be solved by increasing a dilution rate. In addition, since the reaction between the sample and the magnetic marker particles progresses prior to dilution, almost no degradation of sensitivity which is caused by dilution occurs. Therefore, a stable, clear pattern can be formed for a serum having a high concentration.

12

Example 5

HBs Antigen Test of a Blood Sample (No. 1)

[A] Preparation of Anti-HBs Antibody Coated Microplate

An anti-HBs antibody coated microplate was prepared following the same procedures as in Example 3.

[B] Preparation of Anti-HBs Antibody Sensitized Magnetic Latex Beads

A magnetic latex bead solution (concentration: 0.4%) as in Example 1 was prepared using a 0.1M glycine buffer solution of pH 8.3. A rabbit anti-HBs antibody solution (concentration: 1 $\mu$g/m$\ell$) was prepared by dissolving the antigen purified with an affinity column into a glycine buffer solution as described in Example 1. 2 m$\ell$ of the magnetic latex bead solution were mixed with 2 m$\ell$ of the rabbit anti-HBs antibody solution and incubated therewith at 37°C for an hour to sensitize the magnetic latex beads with the HBs antibody. The sensitized magnetic latex beads were washed three times with 2 m$\ell$ (each time) of a 0.1M glycine buffer solution of pH 8.3 containing a 1% BSA. The latex beads were reacted in the same 2-m$\ell$ buffer solution at 37°C for 30 minutes to perform blocking. In addition, the beads were washed three times with 2 m$\ell$ (each time) of a 0.01M PBS containing a 0.01% sodium N-lauroyl sarcosinate as a surfactant available from Nikko Chemicals K.K. and a 0.1% BSA. 4 m$\ell$ of the 0.01M BPS were added to the beads, and the beads were preserved.

[C] Detection of HBs Antigen

An HBs antigen positive blood sample added with an anticoagulant was prepared. An HBs antigen (Midori Juji K.K.) having a subtype of ad was added to this blood sample to prepare a positive sample. The blood sample with no additives was used as a negative sample.

The 0.01M PBS of pH 7.5 containing a 0.01% sodium N-lauroyl sarcosinate and a 5% dextran (molecular weight: 200,000 to 300,000; available from Wako Junyaku K.K.) was added to each well of the anti-HBs antibody coated microplate prepared in [A] in an amount of 100 $\mu\ell$/well. 5 $\mu\ell$ of the positive or negative sample were added to each well, and 25 $\mu\ell$ of the anti-HBs antibody sensitized latex bead solution prepared in [B] were added thereto and were reacted at room temperature for 5 minutes.

A magnetic field from the magnets was applied to the microplate as in Example 1 to precipitate the anti-HBs antibody sensitized latex beads on the bottom surfaces of the wells. The distribution patterns formed by sedimentation can be observed from the lower direction to detect and confirm the presence of the HBs antibody.

Example 6

A, B, and O Blood Typing

[A] Preparation of Group A, B, and O Erythrocyte Coated Microplate

A microplate with a U-shaped bottom (Manufacture No. 464,394 available from NUNC Corp.) was prepared. 100 $\mu\ell$ of a wheat germ lectin (WGA available from Seikagaku Kogyo K.K.) prepared to have a concentration of 10 $\mu$g/m$\ell$ by using 0.01M phosphate buffer solution (PBS) of pH 7.0. The WGA solution were added to each well of the microplate and were treated at room temperature for 30 minutes. Each well was washed five times by using 300 $\mu\ell$ of the 0.0IM PBS of pH 7.0 each time. The microplate was then dried at room temperature to obtain a WGA-treated plate.

A 0.7% physiologial saline suspension of Group A erythrocytes was added to each well of the WGA-treated plate in an amount of 25 $\mu\ell$/well and was left to stand at room temperature for 10 minutes. Each well was washed three times with 200 $\mu\ell$ (each time) of a physiological saline to prepare a Group A erythrocyte coated microplate. Similarly, Group B and O erythrocyte coated microplates were prepared.

[B] Preparation of Antibody Sensitized Magnetic Latex Beads

A magnetic latex bead solution (a solution diluted 50 times) as in Example 1 was prepared by using a 0.1M glycine buffer solution of pH 8.5. A solution (concentration: 20$\mu$g/m$\ell$) of an anti-human IgM antibody available from KPL Corp. was prepared by using a glycine buffer solution as in Example 1. 500 $\mu\ell$ of the magnetic latex bead solution were mixed with the anti-human IgM antibody solution and incubated therewith at room temperature for 30 minutes to sensitize the magnetic latex bead with the anti-human IgM. The sensitized magnetic latex beads were washed twice with 2 m$\ell$ (each time) of a 0.02M glycine buffer solution of pH 8.5 containing a 0.2% BSA and a 0.14M NaCl. Washing was performed by centrifugal washing at 3,000 rpm. Thereafter, the sensitized magnetic latex beads were suspended in the same 2-m$\ell$ buffer solution and were incubated at room temperature for 30 minutes to perform blocking. The resultant beads were cooled and preserved.

[C] A, B, and O Blood Typing

A physiological saline diluted solution of a commercially available anti-A serum (manufacture No. SA960A-1 available from Ortho Corp.) was distributed to some wells of the Group A erythrocyte coated microplate in an amount of 25 $\mu\ell$/well. A physiological saline diluted solution of a commercially available anti-B serum (manufacture No. SB445A-1 available from Ortho Corp.) was distributed to other wells of the Group A erythrocyte coated microplate in an amount of 25 $\mu\ell$/well. The solutions were incubated at 37°C for 10 minutes, and the wells were washed five times with a physiological saline at 200 $\mu\ell$/well. The suspension of the anti-human IgM antibody sensitized magnetic latex beads prepared in [B] was added to each well in an amount of 25 $\mu\ell$/well and were left to stand above the magnets for two minutes, thereby forming sedimentation patterns of the magnetic latex beads and determining positivity ( + ) or negativity (-) from the sedimentation pattern. A test using the Group B and O erythrocyte coated microplates was conducted following the same procedures as described above. Results are shown in Table 5.

## Table 5

| Erythrocyte Blood Group of microplate / Added Antiserum Type | A | B | O |
|---|---|---|---|
| Anti-A Serum | + | – | – |
| Anti-B Serum | – | + | – |

In the assay using the Group A erythrocyte coated microplate, a positive reaction occurred in only the anti-A serum samples. In the assay using the Group B erythrocyte coated microplate, a positive reaction occurred in only the anti-B serum samples. In the assay using the Group O erythrocyte coated microplate, positive reactions occurred in neither the anti-A nor anti-B serum samples.

It is apparent from the above result that Group A, O and B blood samples can be determined.

Example 7

Cross Matching Test

[A] Preparation of Group A and B Erythrocyte Coated Microplate

Group A erythrocyte coated microplate and group B erythrocyte coated microplate were prepared following the same procedures as in Example 6 except that Group A erythrocyle and group B erythrocyte collected from an A-type donor and a B-type donor respectively.

[B] Preparation of Antibody Sensitized Latex Beads

A suspension of anti-human IgM antibody sensitized magnetic latex beads was prepared following the same procedures as in Example 6.

[C] Cross Matching Test

Group A serums collected from A-type donor were added to some wells of the Group A erythrocyte coated plate prepared in [A], and Group B serums collected from B-type donor were added to other wells in an amount of 200 $\mu\ell$/well. The serum samples were incubated at 37°C for 10 minutes, and the wells were washed five times with 200 $\mu\ell$/well (each time) of a physiological saline. The suspension of the anti-human IgM antibody sensitized magnetic latex beads prepared in [B] was added to each well in an amount of 25 $\mu\ell$/well. The wells were left to stand above the magnets to form sedimentation patterns of the magnetic latex beads, thereby determining positivity (+) or negativity (-) from the sedimentation patterns.

A similar test was performed using the Group B erythrocyte coated microplate prepared in [A]. Test results are shown in Table 6.

## Table 6

| Erythrocyte Type of the microplate / Adder Serum | A | B |
|---|---|---|
| Group A | – | + |
| Group B | + | – |

As described above, positive reactions occurred in a combination of the Group A erythrocyte and the Group B serum and a combination of the Group B erythrocyte and the Group A serum. The results show that a cross matching test can be performed. It is possible to wholly determine blood transfusion compatibility by combining the cross matching test with the screening of irregular antibodies in Example 1.

Since detection of irregular antibodies can be performed in Example 1, it is possible to perform a cross compatibility test using anti-human IgG antibody sensitized magnetic particles and anti-human IgM antibody sensitized magnetic particles singly or in a combination.

Example 8

ABO Blood Typing of Coated Erythrocytes using Monochlonal Antibody Sensitized Magnetic Marker Particles

[A] Preparation of Group A, B, and O Erythrocyte Coated Microplates

Group A, B, and O erythrocyte coated microplates were prepared following the same procedures as in Example 6.

[B] Preparation of Antibody Sensitized Latex Beads

A magnetic latex bead solution (a solution diluted 50 times) as in Example 1 was prepared by using a 0.1M glycine buffer solution of pH 8.5. A solution (concentration: 20 $\mu$g/m$\ell$) of an anti-A monochlonal antibody available from Olympus Optical Co., Ltd was prepared by using a glycine buffer solution as in Example 1. 500 u$\ell$ of the magnetic latex bead solution were mixed with 500 $\mu\ell$ of the anti-A monochlonal antibody solution and were incubated at room temperature for 30 minutes to sensitize the magnetic latex beads with the anti-A monochlonal antibody. The sensitized magnetic latex beads were washed twice with 2 m$\ell$ (each time) of a 0.02M glycine buffer solution of pH 8.5 containing a 0.2% BSA and a 0.14% NaC$\ell$. Washing was performed by centrifugal washing at 3,000 rpm. Thereafter, the sensitized latex beads were suspended in the 2-m$\ell$ of same buffer solution and incubated at room temperature for 30 of same minutes to perform blocking. The beads were then cooled and preserved.

Anti-B monochlonal antibody sensitized magnetic latex beads were prepared following the same procedures as described above, except that an anti-B monochlonal antibody available from Olympus Optical Co., Ltd. was used in place of the anti-A monochlonal antibody.

[C] Typing of Group A, B, and O Blood Samples

The suspension of the anti-A monochlonal antibody sensitized magnetic latex beads prepared in [B] was distributed to some wells of the Group A erythrocyte coated microplate prepared in [A] in an amount of 25 $\mu\ell$/well. The suspension of the anti-B monochlonal antibody sensitized magnetic latex beads prepared in [B] was distributed to other wells of the Group A erythrocyte coated microplate prepared in [A] in an

amount of 25 μℓ/well. The samples were placed above the magnets for two minutes to form sedimentation patterns of the magnetic latex beads, thereby determining positivity (+) or negativity (-). Similar tests were performed using the Group B and O erythrocyte coated microplates prepared in [A]. Test results are shown in Table 7.

## Table 7

| Erythrocyte Type of the Microplate Added Serum | A | B | O |
|---|---|---|---|
| Anti-A | + | – | – |
| Anti-B | – | + | – |

As described above, Group A, B and O blood samples can be also determined by using the anti-A monochlonal antibody sensitized magnetic latex beads and the anti-B monochlonal antibody sensitized magnetic latex beads.

Example 9

ABO Blood Typing Using Anti-A Monochlonal Antibody Sensitized Magnetic Marker Particles

[A] Preparation of Anti-A Monochlonal Antibody Sensitized Magnetic Gelatin Beads

200 μℓ of a 5% physiological saline suspension of colored magnetic gelatin beads available from Olympus Optical Co., Ltd. were washed twice by centrifugal washing (2,000 g; five minutes) using 2 mℓ (each time) of a PBS. 2 mℓ of a tannic acid solution (concentration: 20 μg/mℓ) in PBS were added to the colored magnetic gelatin beads and were incubated at 37°C for 30 minutes. The colored magnetic gelatin beads were then washed twice by centrifugal washing (2,000 g; five minutes) using 2 mℓ (each time) of the PBS.

The anti-A monochlonal antibody available from Olympus Optical Co., Ltd. was diluted by a PBS to prepare a solution having a concentration of 10 μg/mℓ. 2 mℓ of this solution were added to the magnetic gelatin beads treated with tannic acid and were incubated at 37°C for 60 minutes to sensitize the magnetic gelatin beads with the anti-A monochlonal antibody. The sensitized magnetic gelatin beads were washed three times by centrifugal washing (1,500 g; five minutes) using 2 mℓ (each time) of a PBS.

The sensitized gelatin beads were suspended in a phosphoric acid-citric acid buffer solution of pH 6 containing a 0.5% BPS and a 0.05% sodium azide and were incubated at 37°C for 30 minutes to perform blocking. The gelatin beads were then cooled and preserved.

[B] Typing of Erythrocyte Antigen

A 0.2% diluted suspension of the Group A erythrocyte was prepared using a phosphoric acid-citric acid isotonic buffer solution of pH 6 containing a 0.5% BSA. 50 μℓ of the suspension of the anti-A monochlonal antibody sensitized magnetic gelatin beads prepared in [A] were added to 50 μℓ of the Group A erythrocyte suspension and were incubated in wells of a microplate having a semispherical bottom surface at 37°C for 10 minutes. In addition, the resultant suspension was left to stand above the magnets for 3 minutes to form sedimentation patterns of the magnetic latex beads, thereby determining positivity (+) or negativity (-) from the sedimentation patterns. Similar tests were performed by using the Group B and O erythrocyte samples in place of the Group A erythrocyte sample. Test results are summarized in Table 8.

16

Table 8

| Beads ╲ Erythrocyte | A | B | O |
|---|---|---|---|
| Monochlonal Anti-A | + | – | – |
| Nonsensitized | – | – | – |

The presence of the Group A erythrocyte was able to be determined within about 13 minutes after the anti-A monochlonal antibody sensitized magnetic gelatin beads were added.

**Claims**

1.  An immunoassay method using magnetic particles on which surface a substance, which can be bonded specifically to or can compete specifically with a substance to be measured, is immobilized (magnetic marker particles) (5), comprising the steps of:
    immunoreacting a sample containing a substance (6) to be detected, with said magnetic marker particles (5) which can be bonded specifically to said substance (6),
    or
    immunoreacting a sample containing a substance (6) to be detected, with said magnetic marker particles (5) which can compete with said substance (6) and a crosslinking substance which bonds to both the substance to be measured (6) and the magnetic marker particles (5); and
    collecting said magnetic marker particles (5) in said reaction solution within a predetermined wall surface region, in a measuring container (2), by applying a magnetic field from a magnet (4) to the reaction solution stored in said measuring container (2), the magnet being located outside the predetermined wall surface region of the measuring container (2),
    **characterized by**
    detecting said substance (6) contained in said sample on the basis of a distribution pattern of said precipitated magnetic marker particles (5) collected in said predetermined wall surface region.

2.  A method according to claim 1, characterized by further comprising the steps of: immobilizing a given substance, which can specifically bond to or compete with the substance to be measured, on at least the predetermined wall surface region of the measuring container (2); and diluting the reaction solution of the sample and the magnetic marker particles.

3.  A method according to claim 1 or 2, characterized in that the predetermined wall surface region is a bottom surface of the measuring container (2).

4.  A method according to any one of claims 1 to 3, characterized in that the predetermined wall surface region is a spherical wall surface.

5.  A method according to any one of claims 1 to 4, characterized in that the predetermined wall surface region is a conical wall surface.

6.  A method according to any one of claims 1 to 5, characterized in that the magnet is a needle-like magnet (8).

7.  A method according to any one of claims 1 to 6, characterized by further comprising the following steps prior to carrying out said immunoreacting step: immobilizing a given substance which can specifically bond to or compete with said substance (6) to be detected, on at least one predetermined wall surface region of said measuring container (2).

8.  A method according to claim 7, characterized in that the predetermined wall surface region is a semispherical wall surface.

9. A method according to claim 7, characterized in that said magnetic marker particles (5) are added to said measuring container (2) after the steps of adding said sample to said measuring container (2) to cause a reaction between said sample and said given substance, removing said sample, and washing said measuring container.

10. A reaction accelerating apparatus for immunoassay comprising:
a supporting base (10) having an upper surface on which a plate having a plurality of reaction containers arranged thereon can be mounted which predetermined wall surface regions are suitable for collecting the magnetic marker particles in a predetermined region; and
a plurality of magnets (12, 14) arranged on said supporting base outside the predetermined wall surface regions of the measuring containers, said magnets being located on positions corresponding to those at which the containers are located when the plate is mounted on said supporting base (10).

11. An apparatus according to claim 10, characterized in that said magnets (12) are in the shape of a needle.

12. An apparatus according to claim 10, characterized in that said magnets (14) are in the shape of a column or prism.

13. An apparatus according to any one of claims 10 to 12, characterized in that male threads are cut in said magnets (14), female threads are cut in said supporting base, and these male and female threads are engaged, such that said magnets (14) can be fixed to the supporting base (10).

14. An apparatus according to any one of claims 10 to 13, characterized in that the edge of said supporting base (10) is raised such that the plate can be reliably supported.

15. An apparatus according to claim 13, characterized in that either of grooves (15) ⊖ and ⊕ is carved on the head of each of said magnets (14).

**Patentansprüche**

1. Immunassayverfahren, welches magnetische Partikel verwendet, auf deren Oberfläche eine Substanz immobilisiert ist (magnetische Markerpartikel) (5), welche spezifisch an eine zu messende Substanz gebunden werden kann oder welche damit spezifisch im Wettbewerb stehen kann, die folgenden Schritte umfassend:
Immunreagieren einer Probe, welche eine zu bestimmende Substanz (6) enthält, mit den magnetischen Markerpartikeln (5), welche spezifisch an die Substanz (6) gebunden werden können, oder
Immunreagieren einer Probe, welche eine zu bestimmende Substanz (6) enthält, mit den magnetischen Markerpartikeln (5), welche mit der Substanz (6) im Wettbewerb stehen können, und mit einer Quervernetzungssubstanz, die sowohl an die zu bestimmende Substanz (6) als auch an die magnetischen Markerpartikel (5) bindet; und
Ansammeln der magnetischen Markerpartikel (5) in der Reaktionslösung innerhalb eines vorbestimmten Wandoberflächenbereiches, in einem Meßbehälter (2) durch Anlegen eines magnetischen Feldes von einem Magneten (4) an die Reaktionslösung, die in dem Meßbehälter (2) enthalten ist, wobei der Magnet an der Außenseite des vorbestimmten Wandoberflächenbereiches des Meßbehälters (2) angeordnet ist,
gekennzeichnet durch
Bestimmen der Substanz (6), die in der Probe enthalten ist auf der Basis eines Verteilungsmusters der präzipierten magnetischen Markerpartikel (5), welche sich in dem vorbestimmten Wandoberflächenbereich angesammelt haben.

2. Verfahren nach Anspruch 1, ferner gekennzeichnet durch die Schritte:
Immobilisieren einer gegebenen Substanz, welche spezifisch an eine zu messende Substanz binden kann oder damit im Wettbewerb stehen kann, an wenigstens dem vorbestimmten Wandoberflächenbereich des Meßbehälters (2); und
Verdünnen der Reaktionslösung der Probe und der magnetischen Markerpartikel.

3. Verfahren nach Anspruch 1 oder 2, dadurch gekennzeichnet, daß der vorbestimmte Wandoberflächenbereich eine Bodenoberfläche des Meßbehälters (2) ist.

4. Verfahren nach irgendeinem der Ansprüche 1 bis 3, dadurch gekennzeichnet, daß der vorbestimmte Wandoberflächenbereich eine kugelförmige Wandoberfläche ist.

5. Verfahren nach irgendeinem der Ansprüche 1 bis 4, dadurch gekennzeichnet, daß der vorbestimmte Wandoberflächenbereich eine konische Wandoberfläche ist.

6. Verfahren nach irgendeinem der Ansprüche 1 bis 5, dadurch gekennzeichnet, daß der Magnet ein nadelartiger Magnet (8) ist.

7. Verfahren nach irgendeinem der Ansprüche 1 bis 6, dadurch gekennzeichnet, daß es ferner die folgenden Schritte vor dem Ausführen des Immunreaktionsschrittes umfaßt:
Immobilisieren einer gegebenen Substanz, die spezifisch an die zu bestimmende Substanz (6) gebunden werden kann oder mit dieser im Wettbewerb stehen kann, auf wenigstens einem vorbestimmten Wandoberflächenbereich des Meßbehälters (2).

8. Verfahren nach Anspruch 7, dadurch gekennzeichnet, daß der vorbestimmte Wandoberflächenbereich eine halbkugelförmige Wandoberfläche ist.

9. Verfahren nach Anspruch 7, dadurch gekennzeichnet, daß die magnetischen Markerpartikel (5) dem Meßbehälter (2) zugegeben werden nach den Schritten der Probenzugabe zu dem Meßbehälter (2), um eine Reaktion zwischen der Probe und der gegebenen Substanz zu bewirken, Entfernen der Probe und Waschen des Meßbehälters.

10. Reaktionsbeschleunigende Vorrichtung für einen Immunassay mit:
einer Trägerbasis (10) mit einer oberen Oberfläche, auf welcher eine Platte mit einer Mehrzahl von Reaktionsbehältern darauf angeordnet ist, kann montiert werden, wobei die vorbestimmten Wandoberflächenbereiche geeignet sind zum Ansammeln der magnetischen Markerpartikel in einem vorbestimmten Bereich; und
eine Mehrzahl von Magneten (12, 14), angeordnet auf der Trägerbasis außerhalb der vorbestimmten Wandoberflächenbereiche der Meßbehälter, wobei die Magneten auf Positionen angeordnet sind, welche jenen entsprechen, bei welchen die Behälter angeordnet sind, wenn die Platte auf der Trägerbasis (10) angeordnet ist.

11. Vorrichtung nach Anspruch 10, dadurch gekennzeichnet, daß die Magnete (12) in Form einer Nadel vorliegen.

12. Vorrichtung nach Anspruch 10, dadurch gekennzeichnet, daß die Magnete (14) die Form einer Säule oder eines Prismas aufweisen.

13. Vorrichtung nach irgendeinem der Ansprüche 10 bis 12, dadurch gekennzeichnet, daß positive Gewinde in die Magneten (14) geschnitten sind, negative Gewinde in die Trägerbasis geschnitten sind und diese positiven und negativen Gewinde derart ineinander eingreifen, daß die Magnete (14) an der Trägerbasis (10) befestigt werden können.

14. Vorrichtung nach irgendeinem der Ansprüche 10 bis 13, dadurch gekennzeichnet, daß die Kante der Trägerbasis (10) derart erhaben ist, daß die Platte zuverlässig getragen werden kann.

15. Vorrichtung nach Anspruch 13, dadurch gekennzeichnet, daß jede der Vertiefungen (15) ⊖ und ⊕ auf den Kopf eines jeden Magneten (14) eingraviert ist.

**Revendications**

1. Procédé de dosage immunologique, utilisant des particules magnétiques à la surface desquelles est immobilisée une substance qui peut se lier spécifiquement à une substance à déterminer ou qui peut entrer en compétition spécifiquement avec une substance à déterminer (particules magnétiques à marqueur) (5), ledit procédé comprenant les étapes consistant à:

- faire réagir par immunoréaction un échantillon contenant une substance (6) à détecter, avec lesdites particules magnétiques à marqueur (5) qui peuvent se lier spécifiquement à ladite substance (6),

    ou bien

- faire réagir par immunoréaction un échantillon contenant une substance (6) à détecter, avec lesdites particules magnétiques à marqueur (5) qui peuvent entrer en compétition avec ladite substance (6), et une substance de réticulation qui se lie à la fois à la substance à déterminer (6) et aux particules magnétiques à marqueur (5), et

- rassembler lesdites particules magnétiques à marqueur (5) dans ladite solution de réaction sur une région prédéterminée de la surface d'une paroi, dans un récipient de mesure (2), en appliquant, à l'aide d'un aimant (4), un champ magnétique à la solution de réaction contenue dans ledit récipient de mesure (2), l'aimant étant situé à l'extérieur de la région prédéterminée de la surface de paroi du récipient de mesure (2), caractérisé en ce que l'on détecte ladite substance (6) contenue dans ledit échantillon en se basant sur le modèle de distribution desdites particules magnétiques à marqueur (5) précipitées, rassemblées sur ladite région prédéterminée de la surface de paroi.

2. Procédé selon la revendication 1, caractérisé en ce qu'il comprend en outre les étapes consistant à:
- immobiliser une substance donnée qui peut se lier spécifiquement à la substance à déterminer ou entrer en compétition avec elle, sur au moins la région prédéterminée de la surface de paroi du récipient de mesure (2); et
- diluer la solution de réaction de l'échantillon et des particules magnétiques à marqueur.

3. Procédé selon la revendication 1 ou 2, caractérisé en ce que la région prédéterminée de la surface de paroi est une surface de fond du récipient de mesure (2).

4. Procédé selon l'une quelconque des revendications 1 à 3, caractérisé en ce que la région prédéterminée de la surface de paroi est une surface de paroi sphérique.

5. Procédé selon l'une quelconque des revendications 1 à 3, caractérisé en ce que la région prédéterminée de la surface de paroi est une surface de paroi conique.

6. Procédé selon l'une quelconque des revendications 1 à 5, caractérisé en ce que l'aimant est un aimant en forme d'aiguille (8).

7. Procédé selon l'une quelconque des revendications 1 à 6, caractérisé en ce qu'il comprend en outre, avant la réalisation de ladite étape d'immunoréaction, l'étape suivante:
- immobiliser une substance donnée qui peut se lier spécifiquement à ladite substance (6) à détecter ou entrer en compétition avec elle, sur au moins une région prédéterminée de la surface de paroi dudit récipient de mesure (2).

8. Procédé selon la revendication 7, caractérisé en ce que la région prédéterminée de la surface de paroi est une surface de paroi hémisphérique.

9. Procédé selon la revendication 7, caractérisé en ce que l'on ajoute lesdites particules magnétiques à marqueur (5) audit récipient de mesure (2) après avoir ajouté ledit échantillon audit récipient de mesure (2) pour provoquer une réaction entre ledit échantillon et ladite substance donnée, éliminé ledit échantillon et lavé ledit récipient de mesure.

10. Appareil pour dosage immunologique, destiné à accélérer la réaction, comprenant:
- une base (10) servant de support, présentant une surface supérieure sur laquelle peut être montée une plaque où sont disposés de multiples récipients à réaction, dont des régions prédéterminées de la surface de paroi conviennent pour la collecte des particules magnétiques à marqueur dans une région prédéterminée, et
- de multiples aimants (12, 14) disposés sur ladite base servant de support, à l'extérieur des régions prédéterminées de la surface de paroi des récipients de mesure, lesdits aimants étant placés en des positions correspondant à celles où sont placés les récipients lorsque la plaque est montée sur ladite base (10) servant de support.

11. Appareil selon la revendication 10, caractérisé en ce que lesdits aimants (12) sont sous la forme d'aiguilles.

12. Appareil selon la revendication 10, caractérisé en ce que lesdits aimants (14) ont la forme d'une colonne ou d'un prisme.

13. Appareil selon l'une quelconque des revendications 10 à 12, caractérisé en ce que des filetages mâles sont formés par découpage dans lesdits aimants (14), des filetages femelles sont formés par découpage dans ladite base servant de support, et ces filetages mâles et ces filetages femelles sont mis en prise de telle sorte que lesdits aimants (14) peuvent être fixés à la base (10) servant de support.

14. Appareil selon l'une quelconque des revendications 10 à 13, caractérisé en ce que le bord de ladite base (10) servant de support est relevé, de sorte que la plaque peut être fixée sur le support de manière sûre.

15. Appareil selon la revendication 13, caractérisé en ce que l'une ou l'autre des rainures (15) ⊖ et ⊕ est gravée sur la tête de chacun desdits aimants (14).

F I G. 1A

F I G. 1B

F I G. 2A

F I G. 3A

F I G. 2B

F I G. 3B

F I G. 4A

F I G. 4B

F I G. 4C

F I G. 5

23

# F I G. 6

# F I G. 7